# EUROPEAN PATENT APPLICATION

(11) **EP 2 942 012 A1**
(43) Date of publication of application: **11.11.2015**
(21) Application number: 14465506.5
(22) Date of filing: 08.05.2014
(51) Int. Cl.: A61B 5/18, B60K 28/06, B60W 40/08, B60W 40/09

(54) **Driver assistance system**

(71) Applicant: Continental Automotive GmbH, 30165 Hannover (DE)
(72) Inventor: Cojocaru, Dragos - Constantin, 700711 Iasi (RO); Capatina, Dinu - Mihai, 700006 Iasi (RO); Miron, Cristian, 700302 Iasi (RO); Constantinescu, Eugen, 700489 Iasi (RO)

(57) **Abstract**

A driver assistance system (100) is presented, comprising a vehicle (102), a database (104) outside the vehicle, and a processing unit (106). The processing unit is configured for determining a health parameter, a driving style parameter, and a mood parameter of a driver and/or passenger of the vehicle. Said health parameter, driving style parameter, and mood parameter are used for determining driver assistance data for assisting the driver of the vehicle (102). At least the health parameter, the driving style parameter, or the mood parameter is determined from data stored on the database (104).

## Description

### Field of the invention

The present invention relates to providing driver assistance in vehicles. In particular, the present invention relates to a driver assistance system, a method for generating driver assistance data, a program element, and a computer-readable medium.

### Background of the invention

There are devices which are capable to monitor the health status of the driver. For example, there are devices for monitoring a blood sugar level of a driver having diabetes. Appropriate measures may be taken when the blood sugar level of the driver is too high.

### Summary of the invention

It is an object of the invention to improve road safety.

This object is solved by the subject-matter of the independent claims. Further embodiments and advantages are set out in the dependent claims, the description, and the figures.

A first aspect of the invention relates to a driver assistance system for a vehicle, which driver assistance system comprises a vehicle, a database outside the vehicle, and a processing unit. The processing unit is configured for determining a health parameter, a driving style parameter, and a mood parameter of a driver and/or a passenger of the vehicle. Hereby, at least the health parameter, the driving style parameter, or the mood parameter is determined from data stored in the database. Furthermore, the processing unit is configured for generating driver assistance data for assisting the driver of the vehicle using the health parameter, the driving style parameter, and the mood parameter of the driver and/or the passenger of the vehicle. Hereby, each of the health parameter, driving style parameter, and mood parameter may be a parameter of the driver and/or passenger.

The driver assistance system may also be understood as a human diagnosis system. It may be seen as a gist of the invention to combine a health parameter, a driving style parameter and a mood parameter of the driver and/or the passenger of the vehicle in order to assist the driver of the vehicle. Hereby, at least one parameter is determined from an external database. By monitoring the health, the driving style, and the mood of the driver and/or the passenger and by assisting the driver based on said monitored parameters, road security may further be improved. Moreover, another advantage may be seen in improving the usability of the vehicle, which also has a positive effect on road security, since the driver may be capable to pay more attention to the traffic when the vehicle is more usable.

In other words, the vehicle behaviour may be adapted based on a deep analysis using three different perspectives, the health, the driving style, and the mood of the driver and/or the passenger. This may provide an intelligent adaptation and configuration of the vehicle as well as customization of the vehicle to the driver and/or the passenger. In this way, the vehicle setting may be less dependent on the car manufacturer and on the type of the vehicle. Furthermore, the owner of the vehicle may be flexible in administering his preferences regarding the vehicle. For example, the driving assistance system may be configured for determining the health parameter from data stored in the database. In this way, the system may make a link between already available data from health systems and the vehicle. Moreover, the driver assistance system may also use sensors from the vehicle and/or from computing devices of the driver and/or the passenger at the same time in order to get a unique human diagnosis model. The vehicle may further include a computing device that may be used for small local analysis, for a faster reaction to events of the vehicle, for providing a communication with an internal core network, and for providing a communication via a secure connection to a human diagnosis service. Said secure connection may be provided by an internal communication unit of the vehicle, for example, GSM, WiFi device, Bluetooth device, or by using computing devices connected to the vehicle. Moreover, it has to be noted that also the health and/or the mood of the passenger may have a high priority in order to assist the driver of the vehicle.

The driver assistance system may provide an adaptable car-to-human diagnosis. The vehicle may, for example be a car, a truck or a motorbike. The database may be stored on a computing device that is located outside of the vehicle. A further database may also be integrated in the vehicle. Or, the database may be partly located outside the vehicle and be partly integrated in the vehicle. The database outside the vehicle may be understood as main database. The database in the vehicle may be used for getting instant decisions and may be synchronized with the database outside the vehicle. In other words, the database may be stored on a stationary server. The processing unit may be integrated in the vehicle, may be located outside the vehicle, or may be partly integrated in the vehicle and partly located outside the vehicle. In other words, the processing unit may have a mobile component that is integrated in the vehicle and a stationary component that is located outside the vehicle. This processing unit may be a part of a human diagnosis service (HDS).

The processing unit may be configured to determine the health parameter, the driving style parameter, and the mood parameter using sensors of the vehicle, input devices of the vehicle, mobile devices of the driver and/or the passenger as well as information from the internet. Thereby, each of the health parameter, the driving style parameter, and the mood parameter may describe a condition of the driver and/or the passenger. Hereby, the health parameter may denote a parameter that describes the health condition of the driver and/or the passenger. The mood parameter may describe a mood state and/or a psychological status of the driver and/or the passenger of the vehicle.

The processing unit may be configured for generating the driver assistance data by combining the health parameter, the driving style parameter, and the mood parameter of the driver and/or the passenger of the vehicle. In other words, the health parameter, the driving style parameter, and the mood parameter may be fusioned in order to generate the driver assistance data. It has to be noted that the step of generating the driver assistance data may also include computing driver assistance data. For example, the processing unit may use artificial intelligence algorithms for generating the driver assistance data on the basis of the health parameter, the driving style parameter, and the mood parameter. Said driver assistance data may be used for assisting the driver of the vehicle. For example, the driver assistance data may comprise instructions for adapting a vehicle parameter. In other words, the processing unit may be configured for sending data comprising instructions for adapting vehicle parameters to a control unit of the vehicle. It has to be noted that the driver assistance may be understood in a broad sense. For example, adapting the interior lights or choosing a specific audio output may assist the driver and increase road security. The driver assistance data may be classified in different scenarios. Depending on the determined health parameter, driving style parameter, and/or mood parameter, the processing unit may choose a different scenario.

According to an exemplary embodiment of the invention, the processing unit comprises a central server or a computer cloud. Furthermore, the processing unit is configured for generating driver assistance data for a plurality of vehicles.

In other words, the processing unit comprises at least a component that is located outside the vehicle. The central server may, for example, denote a server on the internet and the computer cloud may denote a distributed computer network. Thus, the driver assistance system comprises at least partly an external processing unit and/or a stationary processing unit. In this way, a processing unit having a high computing power is provided. Thus, the analysis of the determined parameters may be performed by an external Human Diagnosis Service. However, some operations may be carried out by a computing device of the vehicle. For example, small local analysis may be performed by the computing device of the vehicle for providing a fast reaction to events and/or determined parameters. Moreover, the computing device may provide a secure connection to the processing unit and/or the Human Diagnosis Service.

According to another exemplary embodiment of the invention, the processing unit is configured for generating the driving assistance data using a neuronal network, fuzzy logic, a self-learning algorithm, and/or a self-adapting algorithm.

In this way, the processing unit is able to adapt itself to the driver and/or the passenger of the vehicle. In other words, the processing unit gets to know the driver and/or the passenger of the vehicle.

According to another exemplary embodiment of the invention, the processing unit is further configured for determining a security parameter and for generating the driving assistance data using the security parameter.

The security parameter may comprise a parameter for identifying the driver and/or the passenger, a parameter resulting from a retina scan, a voice recognition parameter, a fingerprint parameter and/or another personal parameter.

In this way, the driver assistance system provides an adaptation of the vehicle behaviour based on a deep analysis using four different perspectives, i.e. health, driving style, mood, and security. Thus, the adaptation of the vehicle is defined for the four categories health, driving style, mood, and security. The processing device may also be configured for generating driver assistance data that is part of a security scenario.

According to another exemplary embodiment of the invention, the health parameter is selected from the group consisting of heart parameters, pulse, sugar level, alcohol level, and/or brain parameters. The driving style parameter is selected from the group consisting of driving dynamics, lane departure parameter, number of lane changes, and/or reaction time. The mood parameter is selected from the group consisting of face parameter, eye parameter, and/or social network status.

According to another exemplary embodiment of the invention, the processing unit is configured for determining the health parameter, the driving style parameter, and the mood parameter using a mobile device of the driver and/or the passenger.

Under the mobile device one may, for example, understand a mobile phone, a smartphone, a tablet PC, and/or a notebook. This mobile device may, for example, comprise different sensors such as microphones, camera and/or acceleration sensors. In this way, further possibilities for determining the health parameter, the mood parameter, and the driving style parameter of the driver and/or the passenger are provided.

According to another exemplary embodiment of the invention, the driver assistance system comprises a communication interface for communicating with a social network and for obtaining information related to the driver and/or the passenger from the social network for determining the health parameter and/or the mood parameter.

It has to be noted that the information that is stored in social networks may be valuable for increasing road security. For example, the driving dynamics of the vehicle may be adapted on the basis of the status information of a social network. The velocity of the vehicle may be limited if the driver is in a bad mood and has published a negative status in the social network. In this way the probability of an accident can be reduced.

According to another exemplary embodiment of the invention, the processing unit is configured for generating the driver assistance data for adapting an interior lighting of the vehicle, adapting a multimedia output of the vehicle, generating an audio signal, generating an optical signal, generating a haptic signal, and/or generating an emergency signal. The haptic signal may, for example, denote a vibration of a steering wheel or a seat of the vehicle. The emergency signal may for example be transmitted to an emergency central.

In this way, measures can be taken based on the generated driver assistance data.

Another aspect of the invention relates to a method for generating driver assistance data for a vehicle. The method comprises the step of determining a health parameter, a driving style parameter, and a mood parameter of a driver and/or a passenger of the vehicle. Moreover, the method comprises the step of generating driver assistance data for assisting the driver of the vehicle using the health parameter, the driving style parameter, and the mood parameter of the driver and/or the passenger of the vehicle. Moreover, at least the health parameter, the driving style parameter, or the mood parameter is determined from data stored in a database outside the vehicle.

This method may for example be carried out by the processing unit of a driver assistance system described in the context of the present invention. Therefore, features and advantages that are described with respect to the driving assistance system are also valid with respect to the method.

Another aspect of the invention relates to a program element, which, when it is processed by a processor, instructs the processor to carry out a method that is described in the context of the present invention. The program element may for example be embodied in form of a computer program or an update of a computer program.

Another aspect of the invention relates to a computer-readable medium, on which a program element is stored, which, when it is processed by a processor, instructs the processor to carry out a method that is described in the context of the present invention.

The aspects described above and further aspects, features and advantages of the invention may also be found in the example embodiments which are described in the following with reference to the appended drawings.

### Brief description of the drawings

Exemplary embodiments of the invention will be described in the following drawings. Any reference signs in the claims should not be construed as limiting the scope of the claims. The drawings may be schematic and not true to scale.
Fig. 1 shows a driver assistance system according to an exemplary embodiment of the invention.
Fig. 2 shows a flow-chart of a method according to an exemplary embodiment of the invention.
Fig. 3 shows a driver assistance system according to an exemplary embodiment of the invention.

### Detailed description of exemplary embodiments

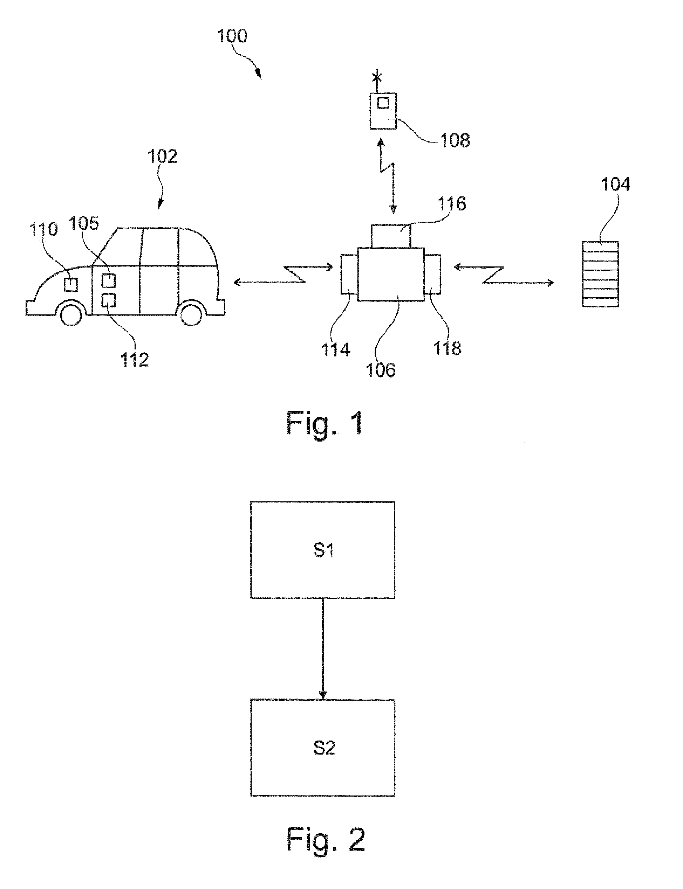

Fig. 1 shows a driver assistance system 100 according to an exemplary embodiment of the invention. The driver assistance system 100 comprises a vehicle 102, a database 104 outside the vehicle 102, and a processing unit 106. The vehicle further comprises a database 105 that is integrated in the vehicle 102. The database 105 is, for example, used for getting instant decisions and is synchronized from time to time with the external database 104. The processing unit is, e.g., part of a Human Diagnosis Service (HDS). The processing unit is configured for determining a health parameter, a driving style parameter and a mood parameter of a driver and/or a passenger of the vehicle 102. At least the health parameter, the driving style parameter, or the mood parameter is determined from data stored in the database 104. Moreover, the processing unit 106 is configured for generating driver assistance data for assisting the driver of the vehicle 102 using the health parameter, the driving style parameter, and the mood parameter of the driver and/or the passenger of the vehicle.

The vehicle 102 further comprises a vehicle control unit 110 and one or more sensors 112. The sensor 112 is configured for determining a health parameter, a driving style parameter, and/or a mood parameter of the driver and/or the passenger of the vehicle 102. The parameter determined by the sensor 112 may be transmitted to the control unit 110, which control unit 110 is configured for transmitting the determined parameter to the processing unit 106. The processing unit 106 comprises first, second, and third interfaces 114, 116 and 118. The first interface 114 is configured for communicating with the control unit 110 of the vehicle 102. The second interface 116 is configured for communicating with a mobile device 108 of the driver and/or the passenger of the vehicle 102. In this way, the health parameter, the driving style parameter, and/or the mood parameter can be determined by the mobile device 108 and be transmitted to the processing unit 106. The third interface 118 of the processing unit 106 is configured for communicating with the database 104. In this way, the processing unit is configured for determining at least the health parameter, the driving style parameter, and/or the mood parameter from data stored in the database 104.

In the following, some use cases are described, which can be carried out by the driver assistance system described in the context of the present invention:
- If the driver of the vehicle 102 has a health issue, which is detected by the driver assistance system, then the driver assistance system can carry out an emergency scenario such that an emergency number is called and useful information about the vehicle 102 and the driver is sent to an emergency central, for example a position of the vehicle 102 and a sugar level in the blood of the driver. Moreover, the vehicle 102 may slow down and stop at the side of the road with activated emergency lights.
- If a passenger of the vehicle 102 has a health issue, which is detected by the driver assistance system, then the driver assistance system carries out an emergency scenario which asks the driver to confirm calling an emergency number and sending useful information about the vehicle and the passenger to an emergency central. Moreover, the driving assistance system instructs the driver and/or the passenger on what to do as a next step in order to improve the health status of the passenger and/or to save the life of the passenger. Moreover, the driver assistance system indicates the shortest way to the next hospital.
- The driver assistance system can adapt the navigation to the mood of the driver. For example, the driver assistance system may instruct the infotainment system of the vehicle to choose a longer but calmer route when a negative mood of the driver is detected.
- The driver assistance system may further be configured to choose the music played by the infotainment system based on the mood detected by the driving assistance system.
- The driver assistance system may further be configured to determine if the vehicle 102 is stolen, for example, based on data being determined by the vehicle sensors. In such a case, the driver assistance system carries out a security scenario. For example, a silent alarm is sent to the owner of the vehicle 102 and to the police department. Furthermore, the position of the vehicle may be sent to the owner and/or the police department. In addition, the vehicle may slow down and stop and issue a breakdown call. Alternatively, the driver assistance system may ask the owner of the vehicle 102 to confirm the next actions. For example, the driving assistance system may send a query to the mobile device of the owner of the vehicle 102.
- The driver assistance system may also download driver-specific settings such as chair position, driving mode, music preferences, and/or point of interests from the database when the driver enters the vehicle. In this way, the vehicle is personally configured. This can especially be of advantage for vehicles of rental companies, where the driver may change often.
- Moreover, the driver assistance system may adapt the infotainment system based on a social network status of the driver. In other words, the current mood stored in the database may depend on the social network status. Based on this information, the music of the infotainment system and points of interests in the navigation route may be set accordingly.
- The driver assistance system may also limit the speed of the vehicle 102 to 80 km/h if tiredness of the driver is detected. Moreover, the driver assistance system may limit the operating region of the vehicle to a certain geographical unit. If tiredness of the driver is detected, a scenario may be carried out in which the vehicle sends warning signals to the driver and in which the vehicle may eventually slow down or even stop.
- The driver assistance system may also adapt the infotainment system based on a picture recorded by a mobile device of the driver and/or the passenger.

Fig. 2 shows a flow-chart of a method according to an exemplary embodiment of the invention. The method comprises the step S1 of determining a health parameter, a driving style parameter, and a mood parameter of a driver and/or a passenger of the vehicle. Furthermore, the method comprises the step S2 of generating driver assistance data for assisting the driver of the vehicle using the health parameter, the driving style parameter, and the mood parameter of the driver and/or the passenger of the vehicle, wherein at least the health parameter, the driving style parameter, or the mood parameter is determined from data stored in a database outside the vehicle.

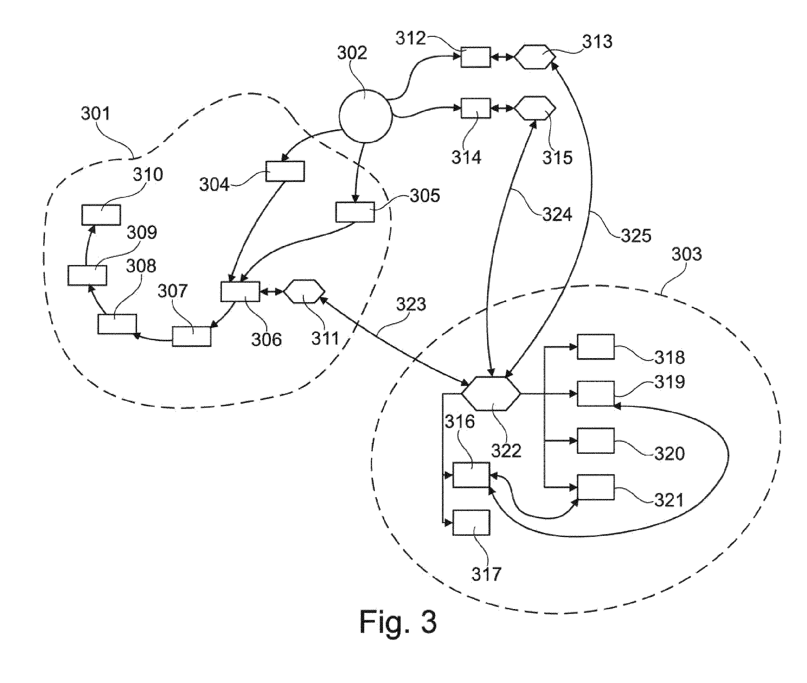

Fig. 3 shows a schematic illustration of a driver assistance system according to an exemplary embodiment of the invention.

The driver assistance system comprises a vehicle 301 and a processing unit 303, which processing unit 303 may also be denoted as Human Diagnosis Service. The vehicle 301 comprises sensors 304, for example a heart sensor, a pulse sensor, a sugar level sensor, an alcohol level sensor, a brain sensor, and/or a weight sensor. In this way, heart parameters, pulse, sugar level, alcohol level, and/or brain parameters may be determined. Moreover, the vehicle includes input devices 305, for example a touch screen, a buttons and/or a keyboard, a joystick, a gesture recognition device, a speech recognition device, a brain sensor, and/or an eye view sensor. The information from the sensors 304 and the input devices is transmitted to a vehicle computing device 306. The driver and/or passenger 302 can interact with the vehicle computing device using different sensors 304 and/or input devices 305. Furthermore, the vehicle computing device 306 comprises a human diagnosis service plug-in 311 which is based on a human diagnosis service API. This human diagnosis plug-in is, for example, incorporated in a browser.

The vehicle computing device is configured for generating events and/or commands for changing the vehicle behaviour 307, these events or commands 307 can be transmitted through the vehicle network 308, for example a CAN, MOST, LAN, Ethernet, etc. In other words, the vehicle network 308 connects different components of the vehicle 301. In particular, the vehicle network 308 connects the vehicle computing device 306 with other vehicle components such that commands and events from the human diagnosis service plug-in are transmitted to the different components of the vehicle. The vehicle network 308 may transmit the events and/or commands to vehicle devices 310 such as power trains, lights, suspension, and/or other vehicle components in order to change the vehicle behaviour.

The processing unit 303 or Human Diagnosis Service (HDS) comprises a service system 318, for example a computer cloud or cloud service. The service system is scalable and fast enough to fulfil the automotive requirements. For example, the service system is embodied as a cloud-based service.

Furthermore, the processing unit 303 comprises a database 319, in which personal data of the driver and/or the passenger of the vehicle 301 is stored. The database 319 comprises two main components, a unit for personal data such as name, mood, preferences, settings, areas of interest, and a unit for automotive data, for example speed, acceleration, brake, light, signal to line change timing, direction change, fuel consumption, and so on. Moreover, the database comprises a unit for automotive data of the vehicle 301. This database may also be separated from the processing unit 303. The processing unit further comprises a data synchronization unit 320 for synchronizing data with social networks and health networks. The data synchronization unit 320 may also be an interface for communicating with social networks. For example, the data synchronization unit may include different plug-ins for communicating with social networks.

Moreover, the processing unit 303 comprises a Human Diagnosis Report unit for reporting a health parameter, a driving style parameter, a mood parameter, a personality information and/or security information. Additionally, the processing unit 303 comprises an HDS artificial intelligence unit 316. The processing device uses the artificial intelligence unit 316 for analyzing the data from the database 319. The artificial intelligence unit 316 may be specialized for the four different human diagnosis service categories (health, driving style, mood, security) and may detect specific states such as health diagnostics, city driving style, bad mood, security emergency. The HDS artificial intelligence unit 316 comprises units for statistic analysis, neuronal networks, and/or other learning-based algorithms. Moreover, the HDS artificial intelligence unit 316 is configured for communicating with the Human Diagnosis Report unit 321 and with the database 319. The report unit 321 is configured for generating human diagnosis reports in the four categories health, mood, driving style and security. The report unit may contain any data coming from the human diagnosis service database 319 and any information coming from the artificial intelligence unit 316. Human diagnosis service scenarios are configured to deal with different report data from the report unit for the driver and/or the passenger. There are links between the human diagnosis report and the defined commands and events. Encoded human diagnosis service commands and events are sent to the service application 322. Subsequently, the encoded data is sent to the computing device 311 of the vehicle 301 using a secure connection. Moreover, the processing device 303 comprises a data synchronization unit 320 to synchronize the database 319 with information from social networks and health networks. For example, data may be imported from social networks.

Additionally, the processing unit comprises a HDS interface (API) 317. The processing device further comprises a user interface 317, for example a text user interface, a graphics user interface, a web interface, and/or a speech recognition interface. Moreover, the processing device comprises a software development interface such as an interface API such that the database may be filled from any computing device and from the vehicle computing device 306 via the plug-in 311. Via the HDS interface 317, the health parameter, the driving style parameter, the mood parameter, and/or the security parameter can be entered in the processing unit 303. Moreover, the processing unit 303 comprises a service application 322 which is, for example, a web application, a SOAP, or a stream-sharing application. For example, the service system 318 is running the service application 322.

The driver or passenger 302 may possess mobile devices 312 and 314. The driver and/or the passenger 302 can also access the human diagnosis service respectively the driver assistance system using these mobile devices 312 and 314. The mobile device 312 comprises sensors for determining the driving style, for example, GPS sensors, acceleration sensors, and/or gyro sensors. The mobile devices 312 and 314 comprise a browser application 313 and 315 including a human diagnosis service plug-in which runs on the mobile devices 312 and 314. In this way, the mobile devices 312 and 314 can send data to the processing unit 303. In this way, the analysis of the processing unit 303 can be improved. The mobile device 314 may denote a mobile device without sensors. Both mobile devices 312 and 314 include a human diagnosis service plug-in 313 and 315 for communicating with the service application 322 of the processing unit 303 and/or Human Diagnosis Service (HDS).

The human diagnosis service plug-ins 311, 313 and 315 communicate with the service application 322 of the processing unit 303 via secure connections 323, 324, and 325. The secure connection 323 between the human diagnosis service plug-in 311 and the service application 322 may use embedded devices from the vehicle 301 such as GSM, WiFi, and/or other wireless connections. The secure connection 323 may use mobile devices that are connected to the vehicle, for example, via Bluetooth, WiFi, NFC, or using another connection. The different vehicle components 310 may denote power trains, lights, suspensions, brakes, and so on. These secure connections 323, 324, and 325 are, for example, embodied as GSM, WiFi or other connections. In this way, the mobile devices 312 and 314 may transfer the health parameter, the driving style parameter, the mood parameter and/or the security parameter to the service application 322 of the processing unit 303. The mobile device 312 with sensors can transmit the health parameter, the driving style parameter, the mood parameter, and/or the security parameter to the service application of the processing unit 303.

Other variations to the disclosed embodiments can be understood and affected by those skilled in the art in practising the claimed invention, from a study of the drawings, from the disclosure, and from the appended claims. In the claims, the word "comprising" does not exclude other elements or steps and the indefinite article "a" or "an" does not exclude a plurality. The mere fact that certain measures are recited in mutually dependent claims does not indicate that a combination of these measures cannot be used to advantage. The reference numerals in the claims are not intended to restrict the scope of the claims.

## Claims

1. Driver Assistance System (100) for a vehicle, comprising:
a vehicle (102);
a database (104) outside the vehicle (102);
a processing unit (106);
wherein the processing (106) unit is configured for determining a health parameter, a driving style parameter, and a mood parameter of a driver or a passenger of the vehicle;
wherein at least the health parameter, the driving style parameter, or the mood parameter is determined from data stored in the database; and
wherein the processing unit (106) is configured for generating driver assistance data for assisting the driver of the vehicle using the health parameter, the driving style parameter, and the mood parameter of the driver and/or the passenger of the vehicle.

2. Driver Assistance System (100) according to claim 1,
wherein the processing unit (106) comprises a central server or a computer cloud; and
wherein the processing unit (106) is configured for generating driver assistance data for a plurality of vehicles.

3. Driver Assistance System (100) according to claim 1 or 2,
wherein the processing unit (106) is configured for generating the driving assistance data using a neuronal network, fuzzy logic, a self-learning algorithm, and/or a self-adapting algorithm.

4. Driver Assistance System (100) according to any one of the preceding claims,
wherein the processing unit (106) is further configured for determining a security parameter and for generating the driving assistance data using the security parameter.

5. Driver Assistance System (100) according to any one of the preceding claims,
wherein the health parameter is selected from the group consisting of heart parameters, pulse, sugar level, alcohol level, and/or brain parameters;
wherein the driving style parameter is selected from the group consisting of driving dynamics, lane departure parameter, number of lane changes, and/or reaction time; and
wherein the mood parameter is selected from the group consisting of face parameter, eye parameter, and/or social network status.

6. Driver Assistance System (100) according to any one of the preceding claims,
wherein the processing unit (106) is configured for determining the health parameter, the driving style parameter and the mood parameter using a mobile device of the driver and/or the passenger.

7. Driver Assistance System (100) according to any one of the preceding claims,
wherein the Driver Assistance System (100) comprises a communication interface (320) for communicating with a social network and for obtaining information related to the driver and/or the passenger from the social network for determining the health parameter and/or the mood parameter.

8. Driver Assistance System (100) according to any one of the preceding claims,
wherein the processing unit (106) is configured for generating the driver assistance data for adapting an interior lighting of the vehicle (102), adapting a multimedia output of the vehicle (102), generating an audio signal, generating an optical signal, generating a haptic signal, and/or generating an emergency signal.

9. Method for generating driver assistance data for a vehicle, the method comprising the steps:
determining a health parameter, a driving style parameter, and a mood parameter of a driver or a passenger of the vehicle (S1); and
generating driver assistance data for assisting the driver of the vehicle using the health parameter, the driving style parameter, and the mood parameter of the driver and/or the passenger of the vehicle (S2);
wherein at least the health parameter, the driving style parameter, or the mood parameter is determined from data stored in a database outside the vehicle.

10. Program element, which, when it is processed by a processor, instructs the processor to carry out the method according to claim 11.

11. Computer-readable medium, on which a program element is stored, which, when it is processed by a processor, instructs the processor to carry out the method according to claim 11.
